# EUROPEAN PATENT SPECIFICATION

(11) **EP 3 329 859 B1**
(45) Date of publication and mention of the grant of the patent: **07.10.2020**
(21) Application number: 15899913.6
(22) Date of filing: 31.07.2015
(51) Int. Cl.: A61B 17/00, A61F 9/007

(54) **IRIS DILATING RING FOR CATARACT SURGERY WITHOUT INJECTOR**
IRISDILATATIONSRING FÜR KATARAKTCHIRURGIE OHNE INJEKTOR
ANNEAU DILATATEUR D'IRIS POUR LA CHIRURGIE DE CATARACTE SANS INJECTEUR

(43) Date of publication of application: 06.06.2018
(73) Proprietor: Canabrava, Sergio Félix, Belo Horizonte - MG (BR)
(72) Inventor: Canabrava, Sergio Félix, Belo Horizonte - MG (BR)
(74) Representative: Karcz, Katarzyna
(86) International application number: PCT/BR2015/000119
(87) International publication number: WO 2017/020099

(56) References cited:
- WO-A1-00/32141
- WO-A1-2014/145739
- BR-U- MU8 502 641
- DE-U1- 9 320 127
- US-A- 6 068 643
- US-A1- 2014 276 900
- US-B1- 6 620 098

## Description

The present invention refers to an iris expansion ring which conjugates the functions of iris expansion and pupil dilation on patients that will be subjected to ophthalmic surgeries, and also permits implantation and withdrawal with a single incision. Handling of the present device spares accessory incisions to the ocular globe as well as the use of injector for its insertion, also displaying an alternated indents structure and low thickness that renders the whole implantation process easier.

Eye illnesses affect a considerable proportion of the world population. Advances in technology and ophthalmic medicine have made it possible to prevent, cure and treat some problems affecting the eye which used to be considered untreatable a few years ago. Most common ocular diseases include myopia, astigmatism, hypermetropia, glaucoma and cataract.

Cataract consists of the total or partial opacity in the crystalline lens, the natural lens in the ocular globe, which is responsible for focalizing eye sight either for near or far sight. It is the progressive diminishing of vision, congenital or acquired (this being the most frequent form of the disease). It affects about a half (46.2%) of world population over sixty five years old. It is estimated that around 160 million people in the world are affected by this disease, which is considered the major cause of avoidable blindness.

The curative treatment of cataract is surgical and consists of replacing the opaque crystalline lens for a prosthesis called intraocular lens (IOL).

As of now there are two main forms of dilating the pupil of patients which will undergo cataract surgery. The first one is using instruments called retractor hooks. Such instruments have the drawback of requiring four extra accessory incisions for its implantation during surgery. The other form is the use of an square-shaped expanding ring which requires an injector for implantation, and is currently protected by the copyright request BR 20200150056856. The requirement of an injector is a setback of this technology when compared to the one presented here, since it increases the cost of the product. Moreover, because it requires four support points on subject iris, it is impossible to use on patients presenting coloboma or sectorial loss of the iris due to trauma.

Intending to solve these inconveniences, the present expansion ring was developed, which can be implanted and withdrawn from subjects eye through the same incision used for extracting the cataract and with no need for an injector. Thus dispensing the need for four accessory incisions. The ring presents a predominantly circular shape, with an opening in one of its sections. Its internal diameter 2 is 6.3 mm, and its thickness 3 is 0.6 mm. It is composed of 7 alternated parts which fits to the eye iris, consisting of larger fitting parts (superior structures) in the superior section of the ring, followed by smaller fitting parts (inferior structures) in the inferior section, repeatedly. In each of these fitting parts there is an orifice for handling of the device inside the ocular globe.

Searching on data banks for patented devices the document US6620098 was found, which describes a device for pupil dilation or for keeping it opened, composed of an opening and a portion of body, having at least seven parts for fitting in the iris, and some of these parts may have internal orifices, and an arm, or hook, for fitting in the iris.

Document US6068643 describes a device for dilating and keeping the pupil open which consists of a body displaying first and second terminations. The external peripheral edge of the body have an engaging formation, adapted to engage the peripheral interior edge of an iris to retain the pupil in an expanded status, and at least one positioning arm which extends out from one end of the body in such a manner that it keeps external to the eye.

Document US2014030550 describes an iris expansion device, which includes a body, unitary and non-metallic, of multiple segments, being expandable from a first status to a second status. This second status defines a bigger space than the first, with the body to be defined by a plurality of segments connected by living hinges. In another aspect, an iris expansion device is provided, which includes a multi-segmented body expandable from a first status to a second status of bigger grip.

Document DE9320127 discloses an iris expander 10 shown in FIG. 1 consisting of a material that is elastically deformable to a great extent, preferably of a multiply sterilizable plastic, and has a substantially circular shape. The circle 11 is interrupted at a point 12. The expander consists of just one structure.

Document WO 00/32141 discloses a pupil dilating device for dilating a pupil, the device being generally hook shaped and having a generally "U" shaped body portion comprising superior and inferior flanges 6 and 7 located opposite each other. The iris fits to the space in between the flanges.

In US 20140276900 an iris expander is disclosed having a multi-segmented body defined by a plurality of segments connected by hinges. There is a void space between the outer and inner parts of the ring.

The invention is defined in independent claim 1. Optional features of the invention are defined in the dependent claims. The iris expansion ring according to the present i disclosure differs from those presented above by the fact that the parts for fitting in the iris are alternated, being one up-oriented and the other down-oriented, and due to this constructive layout, the thickness of the ring is lower, and thus rendering it able to easily enter the ocular globe, bringing great advantages to the functionality of the ring and making implantation easier since the iris connects like a wave to the ring.

Thus, none of the above mentioned documents discloses a device which conjugates the functions of pupil dilating, as well as allowing its implantation and withdrawal by a single incision.

The device conjugates the functions of expanding the iris and dilating the pupil, being an iris expanding ring to be used in ophthalmic surgeries for removal of cataract and insertion of intraocular lens.

According to the i disclosure an iris expansion ring for cataract surgery without injector is provided, the ring having a predominantly circular shape and displaying an opening at one of its sections sided by hook-shaped support points, characterized in that the ring comprises three inferior structures for fitting to the iris edge, the inferior structures being triangular-shaped and displaying an orifice for ring manipulation, the ring further comprising four superior structures for fitting on top of the iris so that it shall connect like a wave on the ring, the superior structures being rectangular-shaped and displaying an orifice for ring manipulation and the superior structures alternating along the circumference of the ring with the inferior structures, which have a smaller area than the superior structures, wherein the planes containing the superior structures are parallel among themselves, the planes containing the inferior structures are parallel among themselves, and the planes containing the superior structures are parallel to the planes containing the inferior structures, and wherein the superior structures are distanced from the inferior structures to create a socket for iris accommodation, the internal diameter of the ring being 6,3 mm and its thickness being 0,6 mm.

Preferably, the iris expansion ring is suitable for being implanted through the incision made for withdrawal of the cataract, the insertion being started by any of the support points of the ring, the ring being then inserted in the ocular globe with the help of simple tweezers commonly used in cataract surgery, and with the ring inserted, a lens manipulating hook being inserted in each orifice for fitting each of the superior structures and each of the inferior structures to patient's iris until its pupil is fully dilated by the ring, and next the lens manipulating hook being used again to detach from the iris each of the superior structures and each of the inferior structures, and then the ring being removed from the ocular globe to finalize the surgery.

The alternation between the superior and inferior fitting structures brings great advantages to the device's functionality, rendering it easier to implant, since the iris shall connect just like a wave on the ring. Moreover, it allows the ring to display a small thickness and thus facilitates its insertion in the eye.

The present invention can be best understood through the attached figures, in which:

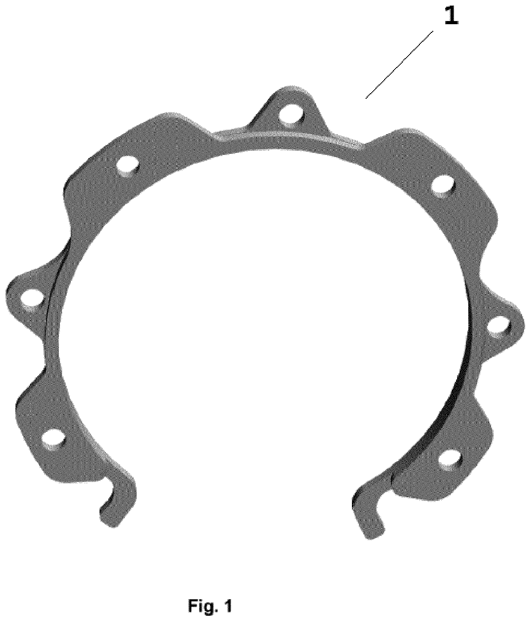

Figure 1 represents the upper view of the ring.

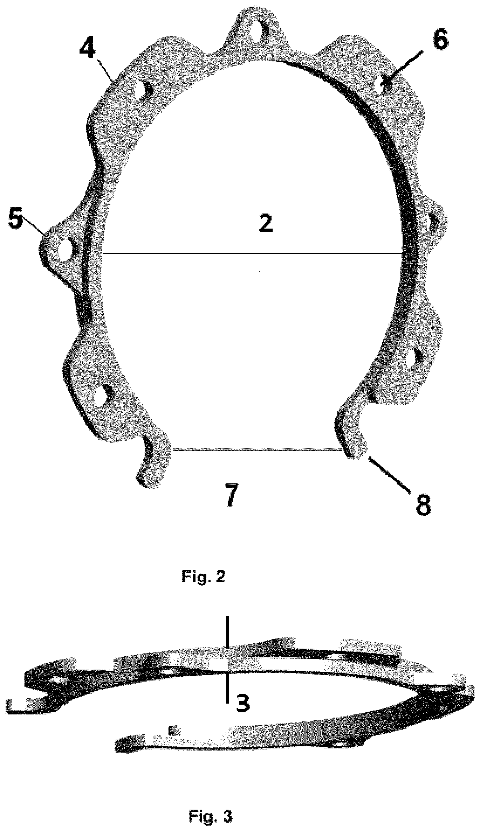

Figure 2 represents a longitudinal view of the ring.
Figure 3 represents a side view of the ring.

The device in comprehends a ring 1 having a predominantly circular shape, presenting an opening 7 in its inferior section, sided by support points 8 which are hook-shaped and situated in both extremities of the ring 1. The ring 1 has a thickness 3 and it comprises three inferior structures 5 which fit to the edge of the iris, displaying triangular shape and an orifice 6 for ring manipulation, and four superior structures 4 alternating along the circumference with the inferior structures 5, which are smaller than the superior ones 4. The planes containing the superior structures 4 are parallel, the same as the planes containing the inferior structures 5. The inferior structures 5 are distanced from the superior structures 4 to create a socket for iris accommodation; the interior diameter of the ring is 6.3, and its thickness is 0.6 mm.

The implantation of the ring 1 can be made through the same incision made for cataract withdrawal. Introduction can be started through any of the open parts (support points 8) of the ring 1.

The ring can be inserted to the ocular globe by simple tweezers commonly utilized in cataract surgery; once inserted inside, a hook must be used for manipulation of the cataract lens; the hook must be inserted in each orifice 6 so as to connect each structure, superior 4 and inferior 5, to the subject iris until the pupil is fully dilated by the ring; next, the surgery can be started and after the implantation of the intraocular lens, the lens manipulation hook is again used to disconnect each of the structures, superior 4 and inferior 5 from the iris, and then proceeding to the withdrawal of the ring 1 from the ocular globe, finalizing the surgery.

## Claims

1. An iris expansion ring (1) for cataract surgery without injector, the ring (1) having a predominantly circular shape and comprising an opening (7) in one of its sections sided by hook-shaped support points (8), wherein the ring (1) comprises three inferior structures (5) for fitting below the iris, the inferior structures (5) being triangular-shaped and comprising an orifice (6) for ring manipulation, the ring (1) further comprising four superior structures (4) for fitting on top of the iris so that i the iris is able to connect like a wave on the ring (1), the superior structures (4) being rectangular-shaped and comprising an orifice (6) for ring (1) manipulation and the superior structures (4) alternating along the circumference of the ring (1) with the inferior structures (5), which have a smaller area than the superior structures (4), wherein a plane contains the superior structures (4) and a plane contains the inferior structures (5), wherein said planes are parallel, and wherein the superior structures (4) are distanced from the inferior structures (5) along a direction perpendicular to the planes to create a socket for iris accommodation, the internal diameter (2) of the ring (1) being 6,3 mm and its thickness (3) being 0,6 mm.

2. The iris expansion ring (1) according to claim 1, suitable for being implanted through the incision made for withdrawal of the cataract by performing the steps of the insertion being started by any of the support points (8) of the ring (1), the ring (1) being then inserted in the ocular globe with the help of simple tweezers commonly used in cataract surgery, and with the ring (1) inserted, a lens manipulating hook being inserted in each orifice (6) for fitting each of the superior structures (4) and each of the inferior structures (5) to patient's iris until its pupil is fully dilated by the ring (1), and next the lens manipulating hook being used again to detach from the iris each of the superior structures (4) and each of the inferior structures (5), and then the ring (1) being removed from the ocular globe to finalize the surgery.

## Patentansprüche

1. Iris-Expansionsring (1) für die Katarakt-Chirurgie ohne Injektor, wobei der Ring (1) eine vorwiegend kreisförmige Form aufweist und
eine Öffnung (7) aufweist, die von hakenförmigen Stützpunkten (8) umgeben ist, wobei der Ring (1) drei untere Strukturen (5) zur Anbringung unter der Iris aufweist, wobei die unteren Strukturen (5) dreieckförmig sind und
eine Mündung (6) zur Ringmanipulation aufweisen, wobei der Ring (1) ferner vier obere Strukturen (4) zum Aufsetzen auf die Iris aufweist, so dass die Iris in der Lage ist, sich wie eine Welle auf dem Ring (1) zu verbinden, wobei die oberen Strukturen (4) rechteckig geformt sind und
eine Mündung (6) zur Manipulation des Rings (1) aufweisen und die oberen Strukturen (4) entlang des Umfangs des Rings (1) mit den unteren Strukturen (5) abwechseln, die eine kleinere Fläche als die oberen Strukturen (4) aufweisen, wobei eine Ebene die oberen Strukturen (4) enthält und eine Ebene die unteren Strukturen (5) enthält, wobei die Ebenen parallel sind,
und wobei
die oberen Strukturen (4) von den unteren Strukturen (5) entlang einer Richtung senkrecht zu den Ebenen beabstandet sind, um eine Fassung für die Irisanpassung zu schaffen, wobei der Innendurchmesser (2) des Rings (1) 6,3 mm und seine Dicke (3) 0,6 mm beträgt.

2. Der Iris-Expansionsring (1) nach Anspruch 1, geeignet für die Implantation durch den Einschnitt, der für die Entfernung der Katarakt gemacht wurde, indem die Schritte des Einführens durch einen der Stützpunkte (8) des Rings (1) begonnen werden, wobei der Ring (1) dann mit Hilfe einer einfachen Pinzette, die üblicherweise in der Kataraktchirurgie verwendet wird, und mit dem eingeführten Ring (1) in den Augapfel eingeführt wird, ein Linsenmanipulationshaken in jede Mündung (6) eingeführt wird, um jede der oberen Strukturen (4) und jede der unteren Strukturen (5) an die Iris des Patienten anzupassen, bis ihre Pupille durch den Ring (1) vollständig geweitet ist, und als nächstes der Linsenmanipulationshaken wieder verwendet wird, um jede der oberen Strukturen (4) und jede der unteren Strukturen (5) von der Iris zu lösen, und dann der Ring (1) vom Augapfel entfernt wird, um die Operation abzuschließen.

## Revendications

1. Un anneau d'expansion d'iris (1) pour la chirurgie de la cataracte sans injecteur, l'anneau (1) ayant une forme principalement circulaire et comprenant
une ouverture (7) dans l'une de ses sections bordée par des points de support en forme de crochet (8), où l'anneau (1) comprend trois structures inférieures (5) pour montage sous l'iris, les structures inférieures (5) étant de forme triangulaire et comprenant
un orifice (6) pour la manipulation de l'anneau, l'anneau (1) comprenant en outre quatre structures supérieures (4) pour montage sur le dessus de l'iris de sorte que l'iris puisse se connecter comme une onde sur l'anneau (1), les structures supérieures (4) étant de forme rectangulaire et comprenant
un orifice (6) pour la manipulation de l'anneau (1) et les structures supérieures (4) alternant le long de la circonférence de l'anneau (1) avec les structures inférieures (5), qui ont une superficie plus petite que les structures supérieures (4), où un plan contient les structures supérieures (4) et un plan contient les structures inférieures (5), où lesdits plans sont parallèles,
et où
les structures supérieures (4) sont éloignées des structures inférieures (5) selon une direction perpendiculaire aux plans pour créer une douille pour accueillir l'iris, le diamètre interne (2) de l'anneau (1) étant de 6,3 mm et son épaisseur (3) étant de 0,6 mm.

2. L'anneau d'expansion d'iris (1) selon la revendication 1, apte à être implanté à travers l'incision pratiquée pour le retrait de la cataracte en exécutant les étapes de l'insertion démarrée par l'un quelconque des points de support (8) de l'anneau (1), l'anneau (1) étant ensuite inséré dans le globe oculaire à l'aide de simples pinces couramment utilisées en chirurgie de la cataracte, et avec l'anneau (1) inséré, un crochet de manipulation de lentille étant inséré dans chaque orifice (6) pour le montage de chacune des structures supérieures (4) et chacune des structures inférieures (5) à l'iris du patient jusqu'à ce que sa pupille soit entièrement dilatée par l'anneau (1), et ensuite le crochet de manipulation de lentille est à nouveau utilisé pour détacher de l'iris chacune des structures supérieures (4) et chacune des structures inférieures (5), et puis l'anneau (1) étant retiré du globe oculaire pour mettre fin à l'opération.
